(19) Europäisches Patentamt
European Patent Office
Office européen des brevets

(11) Numéro de publication: **0 148 660**
**A1**

(12) # DEMANDE DE BREVET EUROPEEN

(21) Numéro de dépôt: **84402405.9**

(22) Date de dépôt: **26.11.84**

(51) Int. Cl.⁴: **C 07 D 471/04**, A 61 K 31/435 // (C07D471/04, 221:00, 209:00)

(30) Priorité: **06.12.83 FR 8319496**

(43) Date de publication de la demande: **17.07.85 Bulletin 85/29**

(84) Etats contractants désignés: **AT BE CH DE FR GB IT LI LU NL SE**

(71) Demandeur: **Etablissement Public dit: CENTRE NATIONAL DE LA RECHERCHE SCIENTIFIQUE (CNRS), 15, Quai Anatole France, F-75007 Paris (FR)**

(72) Inventeur: **Potier, Pierre, 5, rue La Fontaine, F-78390 Bois D'Arcy (FR)**
Inventeur: **Dodd, Robert, 38, rue de Montmorency, F-75003 Paris (FR)**
Inventeur: **Rossier, Jean-Pierre, 42, Domaine de Montvoisin, Gometz La Ville F-91400 Orsay (FR)**

(74) Mandataire: **Warcoin, Jacques et al, Cabinet Régimbeau 26, avenue Kléber, F-75116 Paris (FR)**

(54) **Dérivés de beta-carbolines, procédé pour leurs préparations et médicament les contenant.**

(57) La présente invention concerne des dérivés de β-carbolines, des procédés pour leurs préparations et des médicaments les contenant.

EP 0 148 660 A1

ACTORUM AG

DERIVES DE β-CARBOLINES, PROCEDE POUR LEURS PREPARATIONS ET MEDICAMENT LES CONTENANT.

La présente invention concerne des composés chimiques de la classe des β-carbolines, des procédés pour leur préparation, et leurs applications à titre de médicaments compte tenu de leur haute affinité et sélectivité pour les récepteurs des benzodiazépines.

Plus particulièrement la présente invention concerne des composés de formule I :

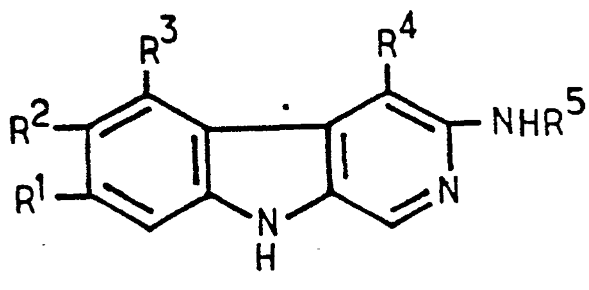

I

dans laquelle :

-$R^1$, $R^2$, $R^3$ et $R^4$ représentent indépendamment un atome d'hydrogène, un radical alkyl inférieur, alcoxy inférieur, un radical benzyloxy ou alcoxyalkyl,

- $R^5$ représente un atome d'hydrogène, un radical alkyl inférieur, un radical alkyl (inférieur) sulfonyle ou un radical $-\underset{\underset{O}{\|}}{C}-R^6$ dans lequel :

-$R^6$ est un atome d'hydrogène, un radical alkyl inférieur, halogéno alkyl (inférieur) ou alcoxyinférieur.

Dans la présente description les termes radicaux alkyl inférieur ou alcoxy inférieur désignent des radicaux en $C_1$ à $C_5$ de préférence $C_1$ à $C_3$ tels que méthyle, éthyle ou méthoxy et éthoxy. Les radicaux halogéno sont le fluor, le chlore, le brome ou l'iode et de préférence le chlore. Les radicaux composés se déduisent des définitions précédentes.

Parmi les composés intéressants, il faut citer en particulier des composés de formule I dans lesquels $R^1$ et $R^2$ représentent un atome d'hydrogène ou un radical méthoxy, $R^4$ représente un atome d'hydrogène, un radical méthyle ou éthyle ou méthoxy méthyl, $R^3$ représente un atome d'hydrogène ou radical benzyloxy, $R^5$ représente un atome d'hydrogène, un radical $C_1$-$C_3$ alkyl, un radical formyl, $C_2$-$C_3$ acyl, $SO_2CH_3$, $C_2$-$C_3$ alcoxycarbonyl.

Plus particulièrement la présente invention concerne les composés choisis parmi :

- l'amino-3-β-carboline,
- la formylamino-3-β-carboline,
- l'acétylamino-3-β-carboline,
- la trifluoroacetylamino-3-β-carboline,
- la méthylsulfonylamino-3-β-carboline,
- l'éthylamino-3-β-carboline,
- l'éthoxycarbonylamino-3-β-carboline.

Ces composés peuvent être synthétisés notamment à partir de l'ester éthylique ou méthylique de l'acide β-carboline-3-carboxylique correspondant de formule II, en suivant le schéma réactionnel suivant.

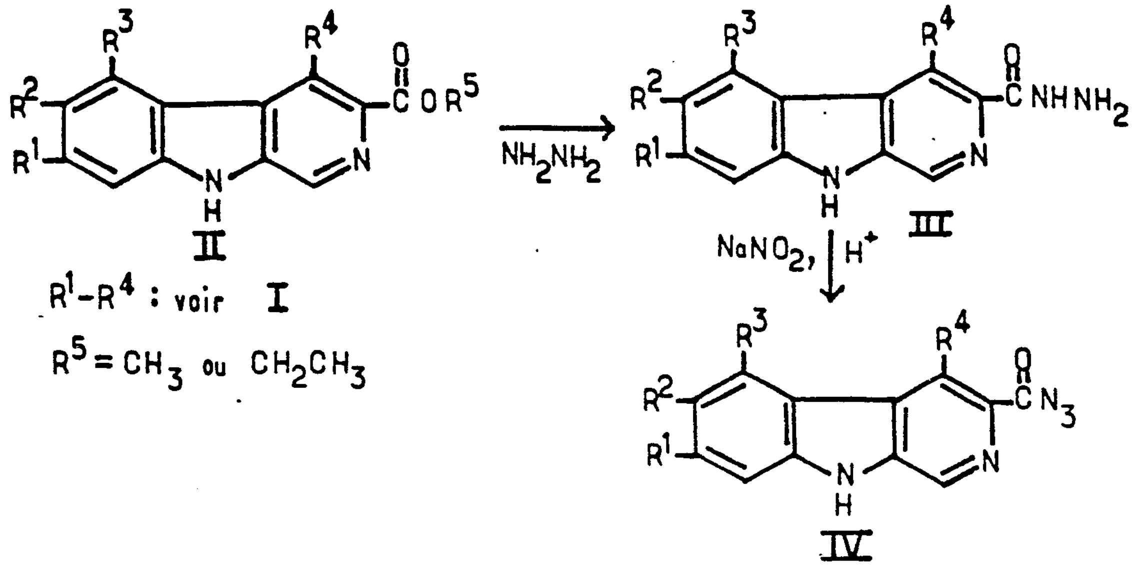


II

$R^1$–$R^4$ : voir I

$R^5 = CH_3$ ou $CH_2CH_3$

III

IV

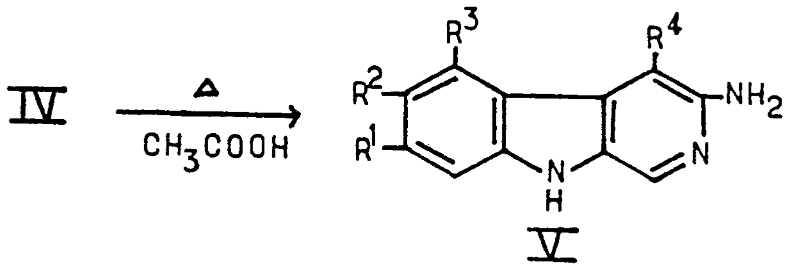


V

Dans ce schéma réactionnel, le composé de formule II est mis en réaction avec de l'hydrazine pour donner le carbohydrazide III qui par réaction du nitrite de sodium en milieu acide donne le carboazide IV qui sert de produit de départ pour toutes les molécules de formule I en vertu du réarrangement de Curtius qui se produit lorsque le composé IV est chauffé :

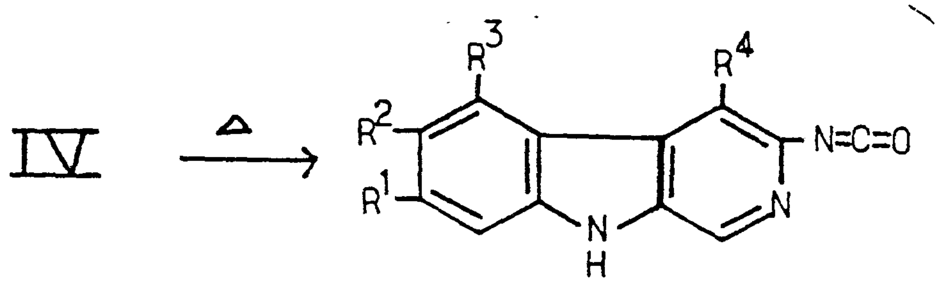

Comme cela est indiqué dans le schéma lorsque l'on porte à ébullition le composé de formule IV dans l'acide acétique, on obtient les amino-3-β-carbolines correspondantes de formule V.

Le composé de formule V peut ensuite être traité pour préparer les différents composés de formule I.

Ainsi on peut porter à ébullition dans un alcool de formule $R^7OH$ (par exemple le méthanol ou l'éthanol) le composé V (ou composé I dans lequel $R^5 = H$) pour obtenir un composé de formule I dans lequel $R^5$ est un radical acyloxy $R^7-O-\underset{O}{\underset{\|}{C}}-$ et $R^7$ est un radical alkyl.

On peut réduire sélectivement un composé de formule VII

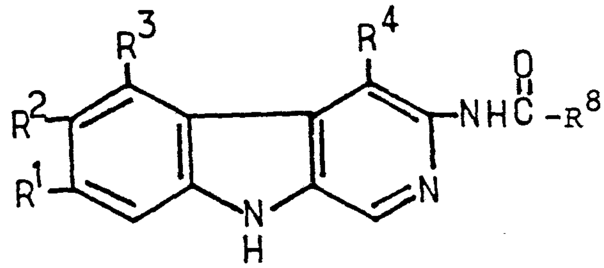

VII

pour obtenir un composé dans lequel $R^5$ est un radical alkyl $-CH_2-R^8$ et $R^8$ est un radical alkyl. Afin d'effectuer cette réduction sélective on utilise de préférence l'alumino hydrure de lithium.

On peut également acyler le composé de formule V avec un agent acylant fixant le radical $R^6-\underset{\underset{O}{\|}}{C}-$ par exemple par action d'un acide carboxylique ou un dérivé d'acide carboxylique tels que halogénure, anhydride ou ester par exemple l'acide formique, l'anhydride acétique, l'anhydride trifluoroacétique.

Pour préparer les composés sulfonylés, on sulfonyle le composé de formule I dans lequel $R^5$ = H avec un agent suflonylant fixant le radical alkyl sulfonyle, de préférence avec l'halogénosulfonate correspondant en particulier le chlorosulfonate.

Les schémas ci-après résument les différentes préparations des composés selon l'invention.

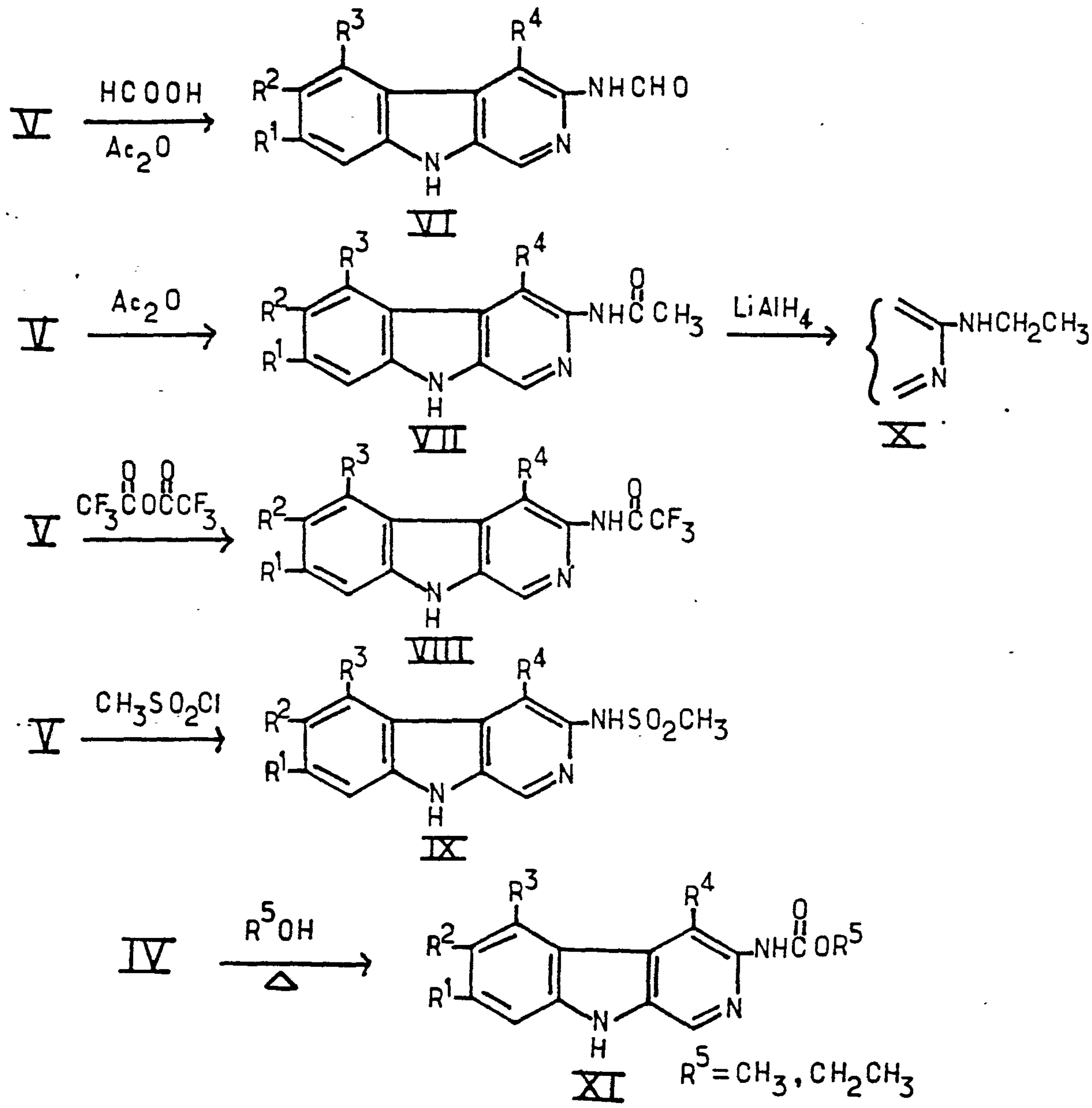


Les composés de départ sont des composés connus ou peuvent être synthétisés par des procédés analogues au procédé décrit dans la technique antérieure.

Les composés selon la présente invention peuvent être utilisés comme médicaments psychotropes, anticonvulsivants psychostimulants, mémotoniques, anxiolytiques, sédatifs, éveillants et antagonistes spécifiques des effets indésirables des benzodiazépines.

Il est bien connu (Braestrup et Squires, Nature, 266, 734 (1977) qu'il existe, dans le système nerveux central des Vertébrés, des sites de fixation (ou "récepteurs") de haute affinité pour les benzodiazépines-1,4 (e.g. Valium®, Librium®). Les effets anxiolytique, sédatif, anticonvulsivant et myorelaxant des benzodiazépines-1, 4 sont le résultat de leur fixation sur ces récepteurs.

Les β-carbolines connues jusqu'à présent se sont révélées être des molécules se liant *in vitro* et *in vivo* avec haute affinité aux récepteurs des benzodiazépines. Les molécules de la classe des benzodiazépines se sont révélées être des médicaments ayant des propriétés anxiolytiques, anticonvulsivantes, sédatives et myorelaxantes. Parmi les effets habituellement considérés comme indésirables, le principal serait l'effet amnésiant des benzodiazépines.

On considère aujourd'hui qu'une molécule se liant au récepteur des benzodiazépines peut engendrer trois types de réponse : ① agonistique, ② antagonistique, ③ agonistique inverse.

① Une réponse agonistique sera comparable à celle obtenue par les benzodiazépines classiques dont un des prototypes est le diazépam (Valium®) soit effet anticonvulsivant, anxiolytique, sédatif, myorelaxant et amnésiant.

② Une réponse antagonistique sera produite par une molécule qui, se liant aux récepteurs, ne produit aucun effet propre. Par contre, ces molécules antagonistes bloquent les effets induits par les agonistes ainsi que ceux induits par les agonistes inverses.

③ Une réponse agonistique inverse sera caractérisée par des effets qui sont inverses de ceux obtenus par un agoniste. Ainsi les agonistes inverses sont convulsivants, anxiogènes, éveillants, psychostimulants. On peut aussi supposer que ces molécules auront un effet mémotonique et myotonique.

Les β-carbolines étudiées jusqu'à présent se placent dans les trois catégories de molécules décrites dans le préambule.

- La molécule de Braestrup (éthoxycarbonyle-3-méthoxy-méthyl-4-benzyloxy-5-β-carboline) est anticonvulsivante et appartiendrait à la classe des agonistes du récepteur des benzodiazépines.

- La propyl β-carboline-3-carboxylate est un antagoniste. Cette molécule bloque les effets anticonvulsivants du diazepam et aussi les effets convulsivants des agonistes inverses.

- La méthyl β-carbolyne-3-carboxylate est un agoniste inverse qui provoque des convulsions, de l'anxiété. Dans cette même catégorie, il y a aussi l'éthyl β-carboline-3-carboxylate dont on a montré l'action éveillante en plus des activités proconvulsivantes et anxiogéniques.

La substitution du carbonyle par un azote sur la chaîne latérale en position 3, permet d'obtenir une nouvelle série de β-carbolines caractérisée :

1. par une plus longue durée d'action, par exemple l'éthylamino-3-β-carboline ou,

2. par une plus grande spécificité pour une des actions des benzodiazépines, ainsi par exemple le méthyl carbamate (méthoxy carbonylamino-3-β-carboline).

Les composés selon la présente invention sont donc utiles à titre de médicaments comme cela a été indiqué précédemment seuls ou en combinaison avec d'autres dérivés notamment des dérivés présentant une activité sur des récepteurs des benzodiazépines, et bien entendu en particulier avec les benzodiazépines elles-mêmes.

D'autres caractéristiques et avantages de la présente invention apparaîtront à la lecture des exemples ci-après :

EXEMPLE 1

Une solution de 2 g de l'ester éthylique de l'acide β-carboline-3-carboxylique dans 20 ml d'éthanol absolu et 10 ml d'hydrate d'hydrazine à 85 % est portée à ébullition pendant 5 heures. Le mélange réactionnel est refroidi et le précipité formé est recueilli par filtration. La recristallisation de ce précipité dans l'éthanol donne 1,5 g de la carbohydrazide-3-β-carboline (III), PF : 289-291°.

EXEMPLE 2

Une suspension de 640 mg de carbohydrazide-3-β-carboline (III) dans l'eau (20 ml) est dissoute par addition d'acide chlorhydrique concentré (0,5 ml). La solution est refroidie à 0° avant l'addition d'une solution de 200 mg de nitrite de sodium dans 1 ml d'eau. Le mélange est maintenu à 0° pendant 30 mn et ensuite rendu alcalin par addition d'une solution saturée de bicarbonate de sodium. Le précipité formé est recueilli par filtration, lavé à l'eau et séché dans un dessicateur pour donner 550 mg de carboazido-3-β-carboline (IV).

EXEMPLE 3

Une suspension de 550 mg de carboazido-3-β-carboline dans 20 ml d'un mélange 1:1 d'acide acétique-eau est portée à ébullition pendant 30 mn. Le mélange réactionnel est refroidi, les solvants sont évaporés sous pression réduite et le résidu cristallisé dans l'éthanol pour donner 300 mg d'amino-3-β-carboline (V), PF : 289-291°.

EXEMPLE 4

Un mélange de 2 ml d'anhydride acétique et 0,8 ml d'acide formique est mis à chauffer à 50-60° pendant 2 heures. La solution est refroidie à 0° et 50 mg d'amino-3-β-carboline (V) sont ajoutés. Au bout d'une heure, un excès de bicarbonate de sodium est ajouté et après 1h50, la solution est extraite deux fois avec l'acétate d'éthyle. Les extraits sont séchés à l'aide de sulfate de sodium, les solvants chassés et le résidu est cristallisé dans un mélange d'alcool-hexane pour donner 40 mg de formylamino-3-β-carboline (VI), PF : 266°.

EXEMPLE 5

Un mélange d'amino-3-β-carboline (50 mg) et d'anhydride acétique (12,5 ml) est agité pendant 2h30mn. Une solution saturée de bicarbonate de sodium (5 ml) est ajouté et l'agitation est maintenue pendant 1h. La solution est ensuite diluée avec 50 ml d'une solution saturée de bicarbonate, extraite 3 fois avec l'acétate d'éthyle, les extraits séchés avec du sulfate de sodium et les solvants chassés, sous pression réduite. Le résidu obtenu est cristallisé dans l'éthanol pour donner 34 mg d'acétylamino-3-β-carboline (VII), PF : 206°.

EXEMPLE 6

Une solution d'amino-3-β-carboline (100 mg) d'anhydride trifluoroacétique (114 mg) et de pyridine (3 ml) est agitée pendant 2 h. Les solvants sont ensuite chassés sous pression réduite et le résidu est purifié par chromatographie sur gel de silice. La trifluoroacétylamino-3-β-carboline (VIII) obtenue est cristallisée dans l'éthanol-hexane (30 mg, PF : 260°).

EXEMPLE 7

Une solution d'amino-3-β-carboline (50 mg), de chlorométhyl sulfonate (32 mg) et de pyridine (1 ml) est agitée pendant 1 h. La solution est diluée avec une solution saturée de bicarbonate de sodium et extraite avec de l'acétate d'éthyle. L'extrait est séché avec du sulfate de sodium, le solvant chassé sous pression réduite et le résidu est cristallisé dans l'éthanol pour donner 40 mg de méthylsulfonylamino-3-β-carboline (IX), PF : 261°.

EXEMPLE 8

A une solution de 300 mg d'acétylamino-3-β-carboline (VII) dans 75 ml de tetrahydrofuranne sec, 67 mg d'hydrure de lithium aluminium sont ajoutés et le mélange est porté à ébullition pendant 2 h. Le mélange est refroidi et 1 ml d'eau saturée de sulfate de sodium est ajouté. Après 2 h, le mélange est filtré, le filtrat est évaporé sous pression réduite et le résidu est purifié par chromatographie sur gel de silice. Ainsi, 200 mg d'éthylamino-3-β-carboline (X) sont obtenus sous forme d'huile.

EXEMPLE 9

Une suspension de 2 g de carboazido-3-β-carboline (IV) dans 150 ml de méthanol anhydre est portée à ébullition pendant 4 h. Le mélange réactionnel est refroidi, concentré sous pression réduite et les cristaux qui se forment sont essorés. Le produit est recristallisé dans le méthanol pour donner 1,4 g de méthoxycarbonylamino-3-β-carboline (XI), PF : 226-228°. De la même façon, l'éthoxycarbonylamino-3-β-carboline est obtenue en utilisant l'éthanol au lieu du méthanol dans la réaction, PF : 222-224°.

EXEMPLE 10 : Activité in vitro des composés

Pour déterminer l'activité pharmacologique des molécules de cette invention, leur affinité pour le récepteur des benzodiazépines a d'abord été étudiée. Cette affinité est déterminée par l'aptitude de la molécule à déplacer une benzodiazépine radioactive (le flunitrazépam tritié) de son récepteur.

Ce pouvoir de déplacement de la molécule est indiqué par son $IC_{50}$, c'est-à-dire la concentration du produit nécessaire pour déplacer 50 % des liaisons spécifiques du $^{3}H$-flunitrazépam avec son récepteur, à partir de tissus préparés du cerveau du Rat.

Pour ces essais *in vitro*, il s'agit d'incuber à 0° pour 60 mn une suspension de tissus provenant du cortex du Rat (≈ 10 mg de tissu par échantillon) dans un milieu tamponné (pH 7,4, 0,05 M Tris-HCl) en présence d'une concentration fixe de $^{3}H$-flunitrazépam (≈ 4 nM, 89 Ci/mmole) et d'une concentration variable du produit ($10^{-3}$ à $10^{-9}$M). La suspension est ensuite filtrée et la radioactivité restant sur le filtre est déterminée par scintillation. L'information obtenue permet de dessiner une courbe de déplacement contre la concentration du produit et de calculer l'$IC_{50}$ du produit.

Pour l'éthylamino-3-β-carboline, un $IC_{50}$ de 4,6 x $10^{-7}$ M est calculé. Par contre, pour la méthoxycarbonylamino-3-β-carboline (la méthylcarbamate), l'$IC_{50}$ est presque dix fois plus faible, ce qui indique pour ce produit une plus haute affinité que pour l'éthylamino-β-carboline. Seulement 80 % du $^{3}H$-flunitrazépam sont déplacés à haute concentration du méthylcarbamate. Ceci est une indication que le méthylcarbamate est sélectif pour une sous-classe des récepteurs des benzodiazépines.

Les résultats sont rassemblés sur le tableau I ci-après.

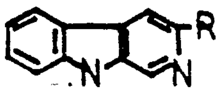

| R | $IC_{50}$ (M) |
|---|---|
| $-CO_2CH_2CH_3$ | $2.0 \times 10^{-9}$ |
| $-NH_2$ | $2.5 \times 10^{-5}$ |
| $-NHCHO$ | $4.7 \times 10^{-6}$ |
| $-NHCOCH_3$ | $4.0 \times 10^{-6}$ |
| $-NHCOCF_3$ | $6.3 \times 10^{-6}$ |
| $-NHCH_2CH_3$ | $4.6 \times 10^{-7}$ |
| $-NHSO_2CH_3$ | $6.2 \times 10^{-5}$ |
| $-NHCO_2CH_3$ | $7.1 \times 10^{-8}$ |
| $-NHCO_2CH_2CH_3$ | $8.0 \times 10^{-8}$ |
| (Flunitrazepam) | $4.0 \times 10^{-9}$ |

<u>Tableau I</u>

EXEMPLE 11 : Activité in vivo de l'éthylamino-3-β-carboline

Cette molécule appartient à la classe des agonistes inverses. Elle provoque des convulsions. Le modèle utilisé est le Babouin photosensible. Sur ce modèle, l'application de stimulation lumineuse intermittente (SLI) par l'intermédiaire d'une lampe stroboscopique provoque des manifestations épileptiques d'amplitude variable. Suivant l'intensité de leur réponse, les animaux sont classés de 0 à + 4.

2 animaux PP 86 et 88 ont été utilisés. Le 86 est + 3 au bout de 30 secondes de SLI, le 5.5.1983. L'administration de l'éthylamino-3-β-carboline (1 mg/kg) réduit cette latence à 10 secondes, 2 minutes après l'injection. 1h30 après l'injection, cette latence est toujours très diminuée (20 secondes). De plus, après l'injection, l'animal fait une crise épileptique tonico-clonique spontanée. Ces résultats sont très différents de ceux de la méthyl-β-carboline dont l'effet convulsivant disparait au bout de 20 minutes chez le Babouin.

Le 88 a montré le même type de résultat. Après 15 secondes de SLI, il est + 3 le 5.5.1983. Après l'administration de 2 mg/kg de N-éthyl carboline, la SLI provoque un stade + 3 au bout de 5 secondes. Cet effet persiste une heure. Après 5 heures, la latence est toujours raccourcie (10 secondes).

EXEMPLE 12 : Activité in vivo du méthyl carbamate

Des expériences de liaison au récepteur des benzodiazépines montrent que ce composé ne déplace que 80 % du $^3$H-flunitrazépam de ces sites de liaison. Cette expérience montrerait qu'il existe au moins 2 sous-sites de liaison. Nous avons émis l'hypothèse que cette définition d'au moins 2 types de sites de liaison du flunitrazépam pour ces récepteurs pouvait avoir un corrélat pharmacologique in vivo. L'occupation d'un des sites entraînerait la sédation induite par le flunitrazépam. L'occupation de l'autre site entraînerait les effets anticonvulsivants du flunitrazépam. Cette hypothèse est confirmée par nos travaux sur le méthyl carbamate.

Dans une première série d'expériences, nous avons remarqué que cette molécule était dénuée d'activité agonistique sur les convulsions induites par le pentylenetetrazol (PTZ).

Protocole expérimental :

40 souris "Swiss", ♂, 30 g recoivent 70 mg/kg s.c. de PTZ. Les souris sont divisées en 4 lots : 1 lot contrôle, 1 lot reçoit 10 mg/kg s.c. de méthyl carbamate, 5 minutes avant le PTZ, 1 lot la même dose de méthyl carbamate 15 minutes avant, et le dernier lot une dose de 1 mg/kg de méthyl carbamate 15 minutes avant. 60 % à 80 % des souris présentent des convulsions dans les 4 lots sans différences statistiquement significatives entre les lots.

Par contre, cette molécule antagonise les effets convulsivants induits par un agoniste inverse de la méthyl-β-carboline.

Protocole expérimental :

100 souris (30 g, ♂) sont traitées par des doses croissantes de méthyl β-carboline de 1 mg à 10 mg/kg s.c. La moitié des souris a été prétraitée par 1 mg/kg de méthyl carbamate s.c. 5 minutes avant l'injection de méthyl β-carboline. Les résultats observés indiquent que le méthyl carbamate antagonise d'une façon compétitive les effets convulsivants de la méthyl β-carboline.

Cette série d'expériences indique que le méthyl β-carbamate se classe dans la série des molécules antagonistiques.

Une série d'expériences complémentaires décrites ci-après indique que l'activité antagonistique serait limitée. En effet, nous avons observé que les effets anticonvulsivants du diazépam ne sont pas bloqués par le méthyl β-carbamate mais que l'action sédative du diazépam est bloquée par le méthyl carbamate. Cet effet est également de longue durée.

Protocole expérimental :

① Absence d'effets antagonistiques sur l'action anticonvulsivante du diazépam :

20 souris (30 g, ♂) ont été traitées avec 70 mg/kg s.c. de PTZ. Une moitié a servi de contrôle ; l'autre moitié a reçu 1 mg/kg s.c. de diazépam 25 minutes avant le PTZ et 10 mg/kg de carbamate 15 minutes avant le PTZ. 80 % des souris contrôlées présentent des convulsions, alors que les souris traitées par le diazépam + méthyl carbamate ne présentent aucun phénomène convulsif.

②. Blocage de la sédation induite par le diazépam :

20 souris (30 g, ♂) ont été entraînées sur le rotarod (bâton de 2,5 cm Ø tournant à 4 rev/mn) pendant 5 minutes. Les souris entraînées seront utilisées si, lors du test précédant l'injection, elles restent 1 minute sans tomber du bâton. Les souris reçoivent toutes 2 mg/kg s.c. de diazépam et sont testées toutes les 10 minutes. Le pourcentage de souris restant 1 minute sur le bâton est indiqué en ordonnée de la figure. Il est clair que le méthyl carbamate à 10 mg/kg s.c. antagonise l'effet sédatif du diazépam.

③. Absence d'effet antagoniste sur les effets anxiolytiques du diazépam :

9 souris ont été entraînées dans un test de conflit décrit par PRADO et al. (Nature 301, 64-66, 1983). En bref les animaux privés de nourriture sont soumis à des séances quotidiennes de 15 minutes dans une boîte de Skinner. Ces sessions comprennent successivement : 5 mn sans conflit (où la souris reçoit une boulette de nourriture chaque fois qu'elle appuie sur la pédale), 5 mn de conflit (où l'appui sur la pédale entraîne à la fois la délivrance d'une boulette et un choc électrique léger), 5 mn sans conflit. Les performances des souris (nombre d'appuis/mn) baissent durant les phases de conflit. Un traitement anxiolytique (diazepam 5 mg/kg s.c.) s'oppose à cette baisse des appuis. L'administration simultanée de méthyl carbamate ne bloque pas cet effet anxiolytique du diazépam.

Des Etudes ultérieures ont permis de mettre en évidence que le méthyl β-carbamate montre une sélectivité pour les récepteurs du cervelet (la sous-classe désignée $BZ_1$) plutôt que pour ceux du cortex et de l'hippocampe (un mélange des sous-classes $BZ_1$ et $BZ_2$) chez la souris. Ceci est démontré par des tests de déplacement du flunitrazépam tritié par le méthyl β-carbamate *in vivo*.

PROTOCOLE EXPERIMENTAL

200 µCi/kg de flunitrazépam tritié sont injectées i.v. chez la souris. Après 10 minutes, une dose connue du méthyl β-carbamate (allant de 2,5 mg/kg jusqu'à 10 mg/kg) est injectée s.c. et 10 minutes plus tard, l'animal est sacrifié. Le cerveau est rapidement disséqué. Le cervelet, le cortex et l'hippocampe sont isolés et broyés séparément et ensuite filtrés. La quantité de radio-activité restant sur le filtre est inversement proportionnelle au pouvoir de déplacement du méthyl β-carbamate.

Les résultats de l'analyse montrent d'abord que le $DE_{50}$ du méthyl β-carbamate (la dose effective pour déplacer 50 % du flunitrazépam tritié) est de 4 mg/kg dans le cervelet, nettement moins élevée que dans le cortex (5 mg/kg) ou l'hippocampe (6 mg/kg).

De plus à une dose de 10 mg/kg, le méthyl β-carbamate déplace presque 100 % du flunitrazépam tritié dans le cervelet mais seulement environ 75 % dans le cortex et l'hippocampe.

Ces résultats sont une indication que le méthyl β-carbamate est sélectif pour les sous-sites de type $BZ_1$ du récepteur des benzodiazépines. Ceci peut expliquer l'action du méthyl β-carbamate en tant qu'antagoniste spécifique des effets sédatifs du diazépam.

REVENDICATIONS

1. Composé de formule :

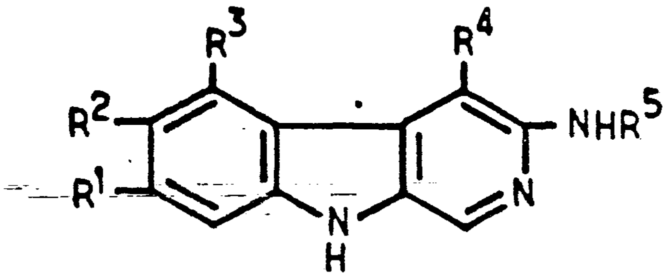

I

dans laquelle :

- $R^1$, $R^2$, $R^3$ et $R^4$ représentent indépendamment un atome d'hydrogène, un radical alkyl inférieur, alcoxy inférieur, un radical benzyloxy ou alcoxyalkyl,

- $R^5$ représente un atome d'hydrogène, un radical alkyl inférieur, un radical alkyl (inférieur) sulfonyle ou un radical $-\underset{\underset{O}{\|}}{C}-R^6$ dans lequel :

- $R^6$ est un atome d'hydrogène, un radical alkyl inférieur, halogéno alkyl (inférieur) ou alcoxyinférieur, à l'exclusion du composé de formule I dans laquelle $R^1$, $R^2$,$R^3$, $R^4$ et $R^5$ représentent un atome d'hydrogène.

2. Composé selon la revendication 1 de formule I

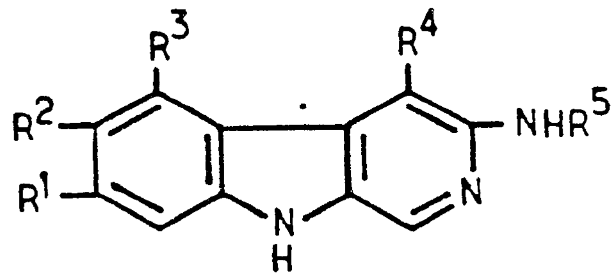

I

dans laquelle :

- $R^1$ et $R^2$ représentent un atome d'hydrogène ou un radical méthoxy,
- $R^4$ représente un atome d'hydrogène, un radical méthyle ou éthyle ou méthoxy méthyl,
- $R^3$ représente un atome d'hydrogène ou un radical benzyloxy,
- $R^5$ représente un atome d'hydrogène, un radical $C_1 - C_3$ alkyl, un radical formyl, $C_2 - C_3$ acyl, $SO_2\ CH_3$, $C_2$-$C_3$ alcoxycarbonyl, à l'exclusion du composé de formule I dans laquelle $R^1$, $R^2$, $R^3$, $R^4$ et $R^5$ représentent un atome d'hydrogène.

3. Un composé choisi parmi :

- la formylamino-3-β-carboline,
- l'acétylamino-3-β-carboline,
- la trifluoroacetylamino-3-β-carboline,
- la méthylsulfonylamino-3-β-carboline,
- l'éthylamino-3-β- carboline,
- l'éthoxycarboxylamino-3-β-carboline.

4. Procédé de préparation des composés selon l'une des revendications 1 à 3 caractérisé en ce que :

a) on porte à ébullition dans l'acide acétique un composé de formule IV

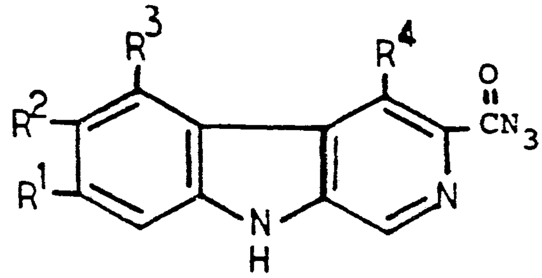

pour obtenir un composé de formule I dans lequel $R^5 = H$,

b) on porte à ébullition dans un alcool de formule $R^7OH$ le composé de formule I dans lequel $R^5 = H$ pour obtenir un composé de formule I dans lequel $R^5$ est un radical acyloxy $R^7 - O - \underset{\underset{O}{\parallel}}{C} -$ et $R^7$ est un radical alkyl,

c) on réduit sélectivement un composé de formule VII

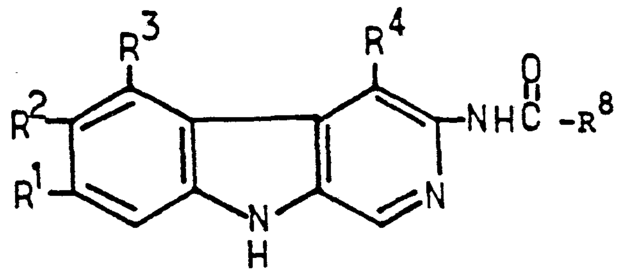

VII

pour obtenir un composé dans lequel $R^5$ est un radical alkyl - $CH_2R^8$ et $R^8$ est un radical alkyl,

d) on acyle le composé de formule I dans lequel $R^5 = H$ avec l'agent acylant fixant le radical $R^6-\underset{\underset{O}{\|}}{C}-$

e) on sulfonyle le composé de formule I dans lequel $R^5 = H$ avec un agent sulfonylant fixant le radical alkyl sulfonyle.

5. Procédé de préparation des composés selon la revendication 4 caractérisé en ce que le composé de formule IV est préparé à partir du composé de formule II.

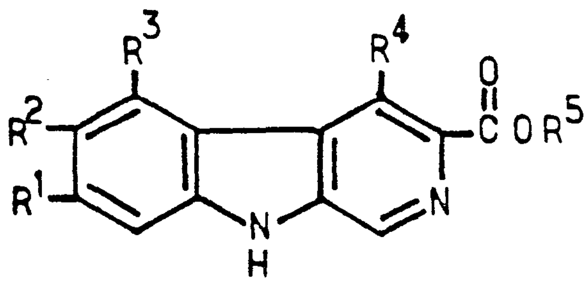

II

par réaction de ce composé avec l'hydrazine pour donner la carbohydrazide de formule III

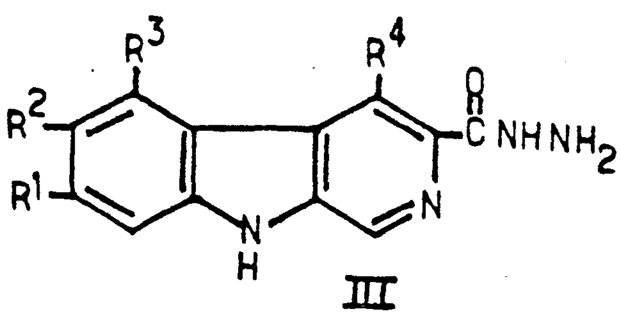

III

que l'on fait réagir avec le nitrite de sodium en milieu acide pour donner le carboazide de formule IV.

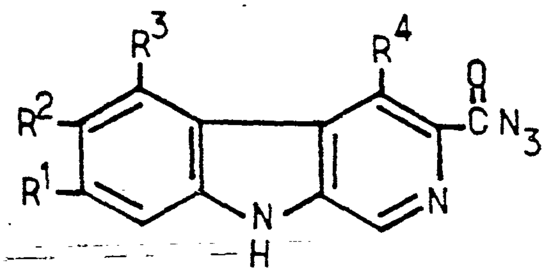

IV

6. Procédé selon l'une des revendications 4 et 5 caractérisé en ce que dans l'étape b) l'alcool utilisé est un alcool en $C_1$ à $C_3$.

7. Procédé selon l'une des revendications 4 et 5 caractérisé en ce que dans l'étape c) la réduction sélective est effectuée avec de l'alumino hydrure de lithium.

8. Procédé selon l'une des revendications 4 et 5 caractérisé en ce que l'agent acylant est un acide carboxylique ou un dérivé d'acide carboxylique.

9. Procédé selon l'une des revendications 4 et 5 caractérisé en ce que l'agent sulfonylant est un halogéno sulfonate.

10. A titre de médicament nouveau un composé de formule :

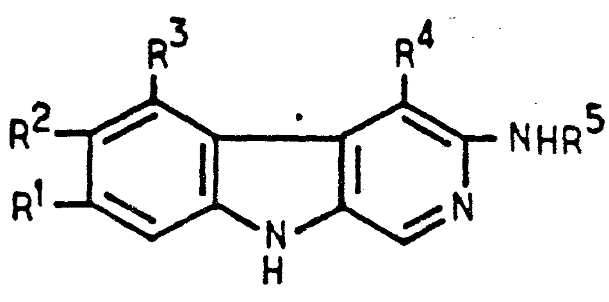

dans laquelle :

- $R^1$, $R^2$, $R^3$, et $R^4$ représentent indépendamment un atome d'hydrogène, un radical alkyl inférieur, alcoxy inférieur, un radical benzyloxy ou alcoxyalkyl,
- $R^5$ représente un atome d'hydrogène, un radical alkyl inférieur, un radical alkyl (inférieur) sulfonyle ou un radical $-\underset{\underset{O}{\|}}{C}-R^6$ dans lequel :

- $R^6$ est un atome d'hydrogène, un radical alkyl inférieur, halogéno alkyl (inférieur) ou alcoxyinférieur,

11. A titre de médicament nouveau un composé de formule :

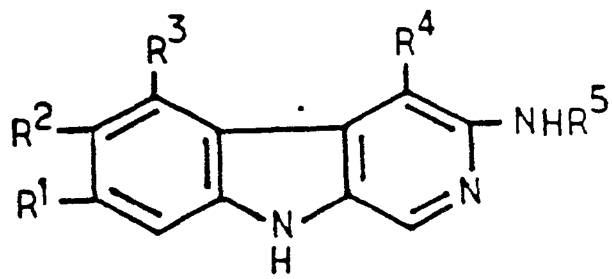

: I

dans laquelle :

- $R^1$ et $R^2$ représentent un atome d'hydrogène ou un radical méthoxy,
- $R^4$ représente un atome d'hydrogène, un radical méthyle ou éthyle ou méthoxy méthyl,
- $R^3$ représente un atome d'hydrogène ou un radical benzyloxy,

- $R^5$ représente un atome d'hydrogène, un radical $C_1$ - $C_3$ alkyl, un radical formyl, $C_2$-$C_3$ acyl, $SO_2CH_3$, $C_2$-$C_3$ alcoxycarbonyl.

12. A titre de médicament nouveau un composé choisi parmi :
- l'amino - 3 - β - carboline,
- la formylamino - 3 -β - carboline,
- l'acétylamino- 3 -β - carboline,
- la trifluoroacetylamino - 3 -β -carboline,
- la méthylsulfonylamino - 3 - β-carboline,
- l'éthylamino - 3 -β -carboline,
- l'éthoxycarboxylamino - 3 -β -carboline.

13. Composition pharmaceutique contenant à titre de principe actif au moins un médicament selon l'une des revendications 10 à 12.

0148660

Office européen des brevets

# RAPPORT DE RECHERCHE EUROPEENNE

Numero de la demande

EP 84 40 2405

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| X | CHEMICAL ABSTRACTS, vol. 96, no. 7, 15 février 1982, page 74, no. 46396u, Columbus, Ohio, US; H. CHANDRA et al.: "Chemical ocelusion of rhesus monkey fallopian tube with 3-amine-9H-pyride(3,4-b)indole" & MED. SCI.: LIBR. COMPEND. 1981, 9(10), 993 * Abrégé * | 10 | C 07 D 471/04<br>A 61 K 31/435//<br>(C 07 D 471/04<br>C 07 D 221:00<br>C 07 D 209:00 ) |
| X | CHEMICAL ABSTRACTS, vol. 98, no. 13, 28 mars 1983, page 190, no. 102389z, Columbus, Ohio, US; HIROKO TOHDA et al.: "Actions of amino-beta-carbolines on induction of sister chromatid exchanges IND: DJJ" & MUTAT. RES. 1983, 116(2), 137-47 * Abrégé * | 10 | DOMAINES TECHNIQUES RECHERCHES (Int. Cl.4) |
| A | EP-A-0 030 254 (SCHERING) * Revendications 1,16 * | 1,10 | C 07 D 471/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 14-03-1985 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82